# EUROPEAN PATENT SPECIFICATION

(11) **EP 0 675 686 B1**
(45) Date of publication and mention of the grant of the patent: **02.07.1997**
(21) Application number: 93900442.0
(22) Date of filing: 25.12.1992
(51) Int. Cl.: A23K 1/16, A23K 1/18

(54) **GRANULAR AGENT FOR RUMINANTS**
GRANULIERTES MITTEL FÜR WIEDERKÄUER
AGENT GRANULAIRE POUR RUMINANTS

(43) Date of publication of application: 11.10.1995
(73) Proprietor: SHOWA DENKO KABUSHIKI KAISHA, Minato-ku, Tokyo 105 (JP)
(72) Inventor: NISHIMURA, Kunio, Eng. Res.Center, Kawasaki-shi, Kanagawa 210 (JP); MORITA, Toshio, Eng. Res. Center, Kawasaki-shi, Kanagawa 210 (JP)
(74) Representative: Strehl Schübel-Hopf Groening & Partner
(86) International application number: JP9201709
(87) International publication number: WO9414335

(56) References cited:
- EP-A- 0 047 142
- EP-A- 0 268 533
- EP-A- 0 276 781
- US-A- 4 277 456
- PATENT ABSTRACTS OF JAPAN vol. 17, no. 409 (C-1091)30 July 1993 & JP,A,05 084 042 (SHOWA DENKO KK) 6 April 1993
- DATABASE WPI Week 8310, Derwent Publications Ltd., London, GB; AN 83-23604K & JP,A,58 015 014 (CENTRAL GLASS KK) 28 January 1983

## Description

### TECHNICAL FIELD

The present invention relates to a granular agent for ruminants and containing a physiologically active substance which agent is used as a feed or feed additive for ruminants. More particularly, the present invention relates to a so-called rumen-bypass granular agent, i.e., an agent in which the physiologically active substance is protected and which is not solubilized in the rumen and is dissolved and absorbed in the abomasum and downstream thereof.

### BACKGROUND ART

It is known that when physiologically active substances such as amino acids and proteins are orally fed to ruminants, the substances are decomposed by microorganisms and enzymes in the rumens to lose their physiological activities inherent thereto.

Therefore, various granular agents for use in feeds or feed additives for ruminants have been proposed which agents are coated with protective substances that protect them from rumen juice and are dissolved and absorbed in a digestive organ including an abomasum and downstream thereof.

For example, Japanese Patent Publication No. 45224/1974 discloses a production method of an amino acid-containing oil capsule in which an amino acid or polypeptide is dispersed in a molten mixture of a fat and fatty oil having a melting point of 40°C or higher and another fat and fatty oil having a melting point of lower than 40°C, and the resulting dispersion is poured into water kept at a temperature of between 20°C and 40°C.

Japanese Patent Publication No. 1057/1981 discloses a granular agent containing a biologically useful substance coated with a matrix comprised by a saturated linear or branched, substituted or unsubstituted aliphatic monocarboxylic acid having at least 14 carbon atoms or salts thereof, or a mixture of such with an unsaturated linear or branched, substituted or unsubstituted aliphatic monocarboxylic acid having at least 14 carbon atoms or salts thereof.

U. S. Patent No. 4,533,557 (Japanese Patent Application Laid-Open No. 175449/1983) discloses a additive composition for ruminant feeds which comprises a physiologically active substance dispersed in a protective matrix containing chitosan and one or more substances selected from the group consisting of a linear or branched, saturated or unsaturated monocarboxylic acid having 14 to 22 carbon atoms, a hardened vegetable oil and a hardened animal oil in which the protective substance is contained in an amount of 50 to 500 parts by weight per 100 parts by weight of the physiologically active substance, and the amount of chitosan in the protective substance is 0.1 to 90 % by weight based on total protective substance.

British Patent Application No. 8501483 (British Patent Application No. 2153199A, Japanese Patent Application Laid-Open No. 168350/1985) discloses granular materials containing a substance useful for a living organism and 20 % by weight or more of a water-insoluble salt of a physiologically acceptable acid which has an acidity weaker than hydrochloric acid (such as tertiary calcium phosphate, secondary calcium phosphate, tertiary magnesium phosphate, zinc phosphate, aluminum phosphate, calcium silicate, calcium pyrophosphate, magnesium carbonate, lead carbonate, cobalt carbonate, etc.), and 10 % by weight or more of at least one substance selected from the group consisting of a linear or branched, saturated or unsaturated monocarboxylic acid having 14 carbon atoms or more or salts thereof, an animal or vegetable fat and fatty oil having a melting point of 40°C or higher, and wax having a melting point of 40°C or higher.

Japanese Patent Application Laid-Open No. 198946/1984 discloses a feed additive containing granules of a physiologically active substance coated with a film which is difficult to dissolve in a neutral or alkaline water but is readily soluble in an acidic water, in which the film is (1) an organic film formed from a film-forming component composed of linear or branched, substituted or unsubstituted, saturated or unsaturated aliphatic monocarboxylic acids having 14 to 22 carbon atoms containing one or more substances selected from one or more of aminocelluloses, polyvinyl-α-aminoacetates and polyvinylamines, or one or more substances selected from the group of aminocelluloses, polyvinylaminoacetates, polyvinylamines and chitosan, and one or more substances selected from the group consisting of hardened vegetable fats and fatty oils, and hardened animal fats and fatty oils, and (2) a film made of one or more inorganic substances selected from metal hydroxides and alkaline metal salts, and a binder, which film is further impregnated or coated with one or more substances selected from the group consisting of aminocelluloses, aminoacetates, vinylamines, aliphatic monocarboxylic acids having 14 to 22 carbon atoms, hardened vegetable fats and fatty oils, hardened animal fats and fatty oils, waxes, beeswaxes, ester gums and water-insoluble binders.

Japanese Patent Application Laid-Open No. 37054/1986 discloses particles of a feed additive containing (A) a physiologically active substance, (B) a substance which is stable under a neutral condition but is disintegrated or dissolved under a weakly acidic condition, and (C) at least one substance selected from linear or branched, saturated or unsaturated monocarboxylic acids having 14 carbon atoms or more or salts thereof, animal and vegetable fats and fatty oils having a melting point of 40°C or higher, and waxes having a melting point of 40°C or higher, in which 20 % or less of particle size from the surface of each granule is occupied by a coating film made of 10 % by weight or more of (B) and 90 % by weight or less of (C).

However, the aforementioned prior arts have problems that the granular agents each have insufficient strengths and insufficient solubilities.

That is, in Japanese Patent Publications Nos. 45224/1974 and 1057/1981, while the disintegration of the film substances was expected to take place in a small intestine or downstream thereof, digestion and absorption of the objective substance tended to occur insufficiently.

Also, in U. S. Patent 4,533,557 (Japanese Patent Application Laid-Open No. 175449/1983) and British Patent Application No. 2153199A (Japanese Patent Application Laid-Open No. 168350/1985), use was made of a substance which is capable of being disintegrated to release a physiologically active substance in an abomasum in order to overcome the aforementioned disadvantages, but the disintegration of such substance is insufficient. On the other hand, increase in the solubility results in the loss of durability in the rumen, and therefore no granular composition has been obtained that has both sufficient durability and sufficient solubility. In addition, among the physiologically active substances, it is impossible to increase the content of those substances which have high solubilities under neutral conditions.

In Japanese Patent Applications Laid-Open Nos. 198946/1984 and 37054/1986, the granules are coated in order to increase the content of the physiologically active substances and improve the durability in the rumen, and in order to increase the solubility (releasability) in the abomasum and downstream thereof, use is made in the coating layer of a solubility controlling substance which is sparingly soluble in a neutral or alkaline water but is readily soluble in an acidic water. In the granular agents disclosed in the aforementioned publications, while the increase in the durability in a rumen depends on the insolubility in water of fats and fatty oils used in the coating, the presence of the solubility controlling substance in the coating layer results in insufficient coating, and provides insufficient means for reducing the occurrence of cracks or the like in the coating layer due to the crystallinity of the fats and fatty oils themselves. For this reason, the increase in the durability of the granular agent in a rumen requires the increase in the coating amount, which makes poor the solubility of the granular agent in an abomasum and downstream thereof.

### DISCLOSURE OF INVENTION

### 1. Summary of the Invention

Therefore, it is an object of the present invention to provide a rumen bypass granular agent which can give a physiologically active substance to ruminants efficiently, that is, a granular agent for ruminants which is not solubilized in the rumen but is dissolved, digested and absorbed in the abomasum and downstream thereof, and to provide a process for producing the same.

As a result of extensive investigations, it has now been found that a granular agent comprised by a core granular agent containing a physiologically active substance coated with a coating layer comprising specified fats and fatty oils, and laminar, tabular crystals of a substance which is sparingly water-soluble under neutral conditions but is readily water-soluble under acidic conditions is free of the above-described disadvantages and suitable as feeds or additive for feeds for ruminants, thus completing the present invention.

That is, the present invention provides a granular agent for ruminants, comprising a core granular agent containing a physiologically active substance coated with a coating layer comprising (a) a first coating material composed of one or more substances selected from the group consisting of a fatty acid having 12 to 22 carbon atoms or ester thereof, an animal or vegetable fat and fatty oil or a hardened animal or vegetable fat and fatty oil, having a melting point of 40°C or higher, and a wax having a melting point of 40°C or higher (hereafter, referred to as "fats and fatty oils"), and (b) a second coating material composed of tabular crystals of a substance being sparingly water-soluble under a neutral condition but readily water-soluble under an acidic condition which have an average particle size of 5 to 30 µm (hereafter, referred to as "tabular crystals"), said coating layer having a laminar structure in which the tabular crystals are arranged in a laminated state.

### 2. Detailed Description of the Invention

Hereafter, the present invention will be described in detail.

The physiologically active substance as used herein refers to any nutrient sources or feeds or drugs containing such nutrient sources so far as they are not in a liquid state.

Examples of the physiologically active substance include the following:
(1) Amino acids and derivatives thereof: Methionine, lysine, tryptophan, threonine, and other amino acids usable as feed additives, N-acylamino acids, hydroxy homologue compounds, and physiologically acceptable salts thereof, such as hydrochlorides, hydrosulfates, ammonium salts, potassium salts, calcium salts, magnesium salts and sodium salts of amino acids.
(2) Vitamins and the derivatives thereof: Vitamin A, vitamin A palmitate, vitamin A acetate, β-carotene vitamin D (D₂, D₃, D₄), vitamin E, menadione sodium bisulfite, vitamin Bs (thiamin, thiamin hydrochloride, riboflavin, nicotinic acid, nicotinic amide, calcium pantothenate choline pantothenate, choline chloride, pyridoxine hydrochloride, cyanocobalamin, biotin, folic acid, p-aminobenzoic acid), vitamin K, vitamin Q, vitamin F, vitamin C, etc.
(3) Enzymes: Protease, amylase, lipase, cellulase, other physiologically effective enzymes, etc.
(4) Animal drugs: Tetracycline type, amino sugar type, and macrolide type antibiotics; other drugs such as polypeptide type and polysaccharide type drugs;
   antiparasitics such a negphon; piperazine salts; etc.
(5) Hormones: Estrogen, stibestrol, hexestrol, tyroprotein, goitradien, etc.
(6) Nutrient sources: Proteins, carbohydrates, etc.
(7) Useful microorganisms: Lactobacillus, Lactobacillus bifidus, and yeasts such as brewers' yeast.
(8) Various minerals:

These physiologically active substances may be used alone or as mixtures of two or more of them.

The content of each physiologically active substance differs in its solubility. Therefore its optimum content can be determined at every physiologically active substance.

The core granular agent containing the physiologically active substance used in the present invention refers to a granular agent prepared from the aforementioned physiologically active substance, one or more optional excipients and a binder and the like.

The core granular agent can be prepared by using any conventional methods known in the art, for example, tumbling granulation, extruding granulation, compression granulation, fluidizing granulation, grinding granulation, agitation granulation, etc.

Upon granulation, a wide variety of excipients and binders usually used in this field of the art can be used. The tabular crystals, which are essential components of the granular agent of the present invention, may be ones which also function as the excipient and the fats and fatty oils may be ones which also function as the binder.

In the present invention, the core granular agent can be coated by using a fumbling granulator or an agitation granulator, more particularly by repeatedly adding the fats and fatty oils usually in a molten state and tabular crystals alternately to the core granular agent containing the physiologically active substance at a temperature by about 5 to 10 °C lower than the melting points of the fats and fatty oils, followed by cooling and solidification to form coating layers repeatedly.

Specific examples of the fats and fatty oils used in the present invention, i.e., one or more of the fatty acid having 12 to 22 carbon atoms or ester thereof, animal or vegetable fat and fatty oil or hardened animal or vegetable fat and fatty oil having a melting point of 40°C or higher, and wax having a melting point of 40°C or higher include, as the fatty acid having 12 to 22 carbon atoms or ester thereof, lauric acid, myristic acid, palmitic acid, stearic acid, behenic acid, oleic acid, rinolic acid, rinoleic acid and esters thereof (glycerol mono-, di- and tri-fatty acid esters, propylene glycol fatty acid esters, sorbitan fatty acid esters, etc.); as the animal and vegetable fats and fatty oils having a melting point of 40°C or higher: palm oil, beef tallow, porcine tallow, etc.; as the hardened animal and vegetable fats and fatty oils: hardened castor oil, hardened colza oil, hardened beef tallow, extremely hardened beef tallow, etc.; and as the wax: bee wax, carnauba wax, Japan wax, etc.

Among the fats and fatty oils, preferred are hardened animal and vegetable fats and fatty oils, with the extremely hardened beef tallow being particularly preferred.

In the present invention, the tabular crystals in the coating layers, i.e., tabular crystals of a substance being sparingly water-soluble under a neutral condition but readily water-soluble under an acidic condition which have an average particle size of 5 to 30 µm, can be prepared by grinding or sifting tabular crystals of the substance which does not dissolve under a neutral condition but dissolves under an acidic condition. The average particle size as used herein refers to average particle size on weight basis measured using a usually used sedimentation particle size distribution analyzer after conversion into spheres.

Specific examples of the tabular crystals of the substance which is sparingly water-soluble under a neutral condition but is readily water-soluble under an acidic condition include tabular crystals of amino acid such as isoleucine, cystine, and methionine, and tabular crystals of inorganic salts such as zinc phosphate octahydrate, and secondary calcium phosphate dihydrate. Among these, tabular crystals of cystine, zinc phosphate octahydrate, and secondary calcium phosphate dihydrate are preferred, with tabular crystals of secondary calcium phosphate dihydrate, or cystine being particularly preferred.

As the excipient used upon granulation other than the tabular crystals which are essential components of the granular agent of the present invention, there can be cited, for example, carbonates such as calcium carbonate, magnesium carbonate, cobalt carbonate, and aluminum carbonate; inorganic salts such as tertiary calcium phosphate, secondary calcium phosphate, tertiary magnesium phosphate, secondary magnesium phosphate, zinc phosphate, aluminum phosphate, calcium silicate, and calcium pyrophosphate; metal oxides such as magnesium oxide, calcium oxide, and silicon dioxide; polysaccharides such as chitosan, chitin, calcium alginate and carrageenan; and synthetic high molecular weight substances such as cellulose derivatives, and polyvinyl derivatives.

The tabular crystals used in the present invention refers to crystals of which proportions of sides are in the range of 1 : 2 : 2 to 1 : 50 : 50 (height : length : depth). Such tabular crystals can be obtained by adjusting the crystallization method. Also, the tabular crystals can be distinguished one from another by X-ray powder diffractometry. For example, in the case of secondary calcium phosphate dihydrate, the tabular crystals refers to those which have a diffraction strength ratio I_{2.94}/I_{3.06} defined as a ratio between interplanar distances of 2.94 (Å) and 3.06 (Å) as measured by X-ray diffractometry (CuKα) is 0.3 or less.

In the case where use is made of a substance other than the tabular crystals of the substance which is sparingly water-soluble under a neutral condition but is readily water-soluble under an acidic condition upon the granulation, there is obtained no laminar structure in the coating layer in which such a substance is present in a laminated state, resulting in that the coated layer becomes brittle, and no sufficient durability in a rumen can be obtained unless the coated amount is increase. However, the increase in the coated amount in order to obtain increased durability results in insufficient solubility (releasability) of the physiologically active substance in the abomasum or downstream thereof.

Even when the tabular crystals are used, those which have average particle sizes of 30 µm or larger suffer from insufficient coating of the tabular crystals with the oils and fats so that no laminar structure can be formed in the coating layers, thus giving a poor durability in the rumen. If the tabular crystals used have an average particle size of 5 µm or smaller, the solubility (releasability) in the abomasum or downstream thereof is poor although a laminar structure is formed in the coating layers.

Proportion of the fats and fatty oils to the tabular crystals in the coating layers is preferably in the range of 3 : 2 to 1 :3 by weight. If the tabular crystals are used in proportions more or less than the above proportion, thus failing to give sufficient effects because of a poor durability in the rumen or a poor solubility in the abomasum and downstream thereof.

While the coating ratio of the core granular agent containing physiologically active substance should be determined depending upon the solubility of the physiologically active substance in the abomasum or downstream thereof, or characteristics of the core granular agent, and performance to be obtained, usually the coating ratio is 5 % by weight or more and 50 % by weight or less, based on the total weight of the granular agent, wherein the coating ratio is defined as the amount by weight of the coating layer to the amount by weight of the granular agent according to the present invention. If the coating ratio is less than 5 % by weight, the durability in the rumen is poor and no sufficient effects can be obtained. On the other hand, if use is made of a large amount of the coating material as much as 50 % by weight or more, the content of the effective ingredient decreases, thus giving insufficient effects.

In the granular agent for feeds for ruminants and additives thereto according to the present invention, obtained by coating the core granular agent composed mainly of physiologically active substance with a coating layer comprising specified fats and fatty oils and tabular crystals of the substance which is sparingly water-soluble under a neutral condition and readily water-soluble under an acidic condition and having an average particle size of 5 to 30 µm, the fats and fatty oils in the coating layers function as a protective substance which increases the durability in the rumen. The aforementioned tabular crystals form a laminar structure in which they are present in a laminated state in the coating layers. The laminar structure strengthens the coating layers to increase not only the durability of the granular agent in the rumen but also the solubility (releasability) in the abomasum or downstream thereof.

The granular agent for ruminants according to the present invention exhibits excellent performance as a rumen-bypass granular agent for ruminants, and can be dissolved to release a physiologically active substance useful as a feed or additive thereto in an abomasum or downstream thereof, thus allowing the physiologically active substance to be digested and absorbed efficiently.

### BEST MODE FOR CARRYING OUT THE INVENTION

The present invention will be described in greater detail by examples and comparative examples. However, the present invention should not be construed as being limited thereto.
(1) Granulation of a core granular agent containing a physiologically active substance:
   (a) Granulation of a core granular agent containing lysine hydrochloride:
      1,500 g of L-lysine hydrochloride and 540 g of extremely hardened beef tallow were granulated using a tumbling granulator with heating to obtain a granular agent of 8 to 24 mesh.
   (b) Granulation of a core granular agent containing anhydrous glucose:
      1,200 g of anhydrous glucose, 75 g of calcium carbonate, 38 g of silicon dioxide and 300 g of extremely hardened beef tallow were granulated using a Henschel mixer with heating to obtain a granular agent of 8 to 24 mesh.
   (c) Granulation of a core granular agent containing magnesium L-ascorbate phosphate:
      1,000 g of magnesium L-ascorbate phosphate and 200 g of extremely hardened beef tallow were granulated using an agitation granulator with heating to obtain a granular agent of 8 to 24 mesh.
   (d) Granulation of a core granular agent containing choline chloride:
      1,000 g of choline chloride, 100 g of calcium carbonate, 100 g of silicon dioxide and 510 g of extremely hardened beef tallow were granulated using an agitation granulator with heating to obtain a granular agent of 8 to 24 mesh.
   (e) Granulation of a core granular agent containing DL-methionine:
      1,500 g of DL-methionine and 340 g of extremely hardened beef tallow were granulated using a spartan extruder with heating to obtain a granular agent of 8 to 24 mesh.
   (f) Granulation of a core granular agent containing L-tryptophan:
      1,500 g of L-tryptophan and 490 g of extremely hardened beef tallow were granulated by using an agitation granulator with heating to obtain a granular of 8 to 24 mesh.
   (g) Granulation of a core granular agent containing nicotinic acid:
      920 g of nicotinic acid, 32 g of calcium carbonate, 64 g of silicon dioxide and 312 g of extremely hardened beef tallow were granulated by using an agitation granulator with heating to obtain a granular agent of 8 to 24 mesh.
   (h) Granulation of a core granular agent containing betaine:
      1360 g of betaine, 250 g of calcium carbonate and 360 g of extremely hardened beef tallow were granulated by using an agitation granulator with heating to obtain a granular agent of 8 to 24 mesh.
   (i) Granulation of a core granular agent containing vitamin C:
      1500 g of vitamin C, 70 g of silicon dioxide and 320 g of extremely hardened beef tallow were granulated by using an agitation granulator with heating to obtain a granular agent of 8 to 24 mesh.
   (j) Granulation of a core granular agent containing glycine and alanine:
      650 g of glycine, 650 g of DL-alanine and 303 g of extremely hardened beef tallow were granulated by using an agitation granulator with heating to obtain a granular agent of 8 to 24 mesh.
(2) Coating of a core granulator containing a physiologically active substance:

### Examples 1 to 3 and Comparative Examples 1 to 5: Granular agent having a coated core granular agent containing lysine hydrochloride

In an agitation granulator were charged 1,500 g of a core granular agent containing lysine hydrochloride and the temperature of the material was kept at 50°C by using main spindle alone of the granulator. Then, using molten extremely hardened beef tallow and eight (8) kinds of coating substances shown in Table 1 (Examples 1 to 3 and Comparative Examples 1 to 5) , aliquots of the core granular agent were coated, respectively, to a coating ratio of 33 % by weight based on the total weight of the resulting granules. The extremely hardened beef tallow and each of the coating substances shown in Table 1 were used in a proportion of 2 : 3 (by weight), and added alternately.

**Table 1**

| Coating Substance | |
|---|---|
| Example 1 | Tabular crystals of secondary calcium phosphate dihydrate (average particle diameter: 10 µm) |
| Example 2 | Tabular crystals of L-cystine (average particle size: 10 µm) |
| Example 3 | Tabular crystals of DL-methionine (average particle size: 10 µm) |
| Comparative Example 1 | Prismatic crystals of secondary calcium phosphate dihydrate (average particle diameter: 10 µm) |
| Comparative Example 2 | Tertiary calcium phosphate (amorphous) |
| Comparative Example 3 | Calcium carbonate (amorphous) |
| Comparative Example 4 | Tabular crystals of secondary calcium phosphate dihydrate (average particle diameter: 35 µm) |
| Comparative Example 5 | Tabular crystals of secondary calcium phosphate dihydrate (average particle diameter: 2 µm) |

Observation of the cross sections of the granular agents of Example 1 and Comparative Example 1 by scanning electron micrography (magnification: ×500) revealed that the granular agent of Example 1 using tabular crystals of secondary calcium phosphate dihydrate having an average particle size of 10 µm had a laminar structure in the coating layer in which the tabular crystals were arranged in a laminated state while in the granular agent of Comparative Example 1 using prismatic crystals no such laminar structure was found.

Also, scanning electron micrographic observation of the cross sections of the granular agents obtained in Examples 2 and 3 indicated that the respective coating layers of these granular agents showed laminar structures similar to the laminar structure found in the coating layer of the granular agent of Example 1.

Dissolution tests of the resulting granular agents were conducted in a mimic-rumen solution (in vitro), in a rumen of sheep (in vivo), in a mimic-abomasum solution (in vitro), respectively.

Test solutions and test method used are as follows.

### (a) Dissolution test method

One gram (1 g) each of the granular agents containing physiologically active substances prepared previously was immersed in 50 ml each of the mimic-rumen solution and the mimic-abomasum solution, respectively, and the resulting mixtures were shaken at 39°C for 18 hours. Thereafter, the physiologically active substances in the test solutions were quantitatively determined by high performance liquid chromatography, and dissolution rates thereof were obtained.

### (b) Composition of the mimic-rumen solution

6.3 g or primary sodium phosphate dihydrate, 30.5 g of secondary sodium phosphate, 6.2 g of primary potassium phosphate, 0.6 g of potassium chloride, 7.9 g of sodium chloride, 0.3 g of calcium chloride, 0.3 g of magnesium chloride, and 31.5 g of sodium bicarbonate were dissolved in water to make 3 liters. The pH of the resulting solution was 7.1.

### (c) Composition of the mimic-abomasum solution

20.5 g of sodium acetate, 8.8 g of sodium chloride, and 3 ml of 0.1 vol.% ethanol were dissolved in water to make 3 liters with adjusting pH to 2.

### (d) Test method for solubility in the rumen of sheep

One gram (1 g) of the granular agent previously prepared was put in a 200-mesh nylon bag, which was immersed for 24 hours in the rumen of rumen-fistulated-sheep. The physiologically active substance which remained in the granular agent was quantitatively determined by high performance liquid chromatography to obtain remaining rates which were converted to dissolution rates (%).

Results of the dissolution tests are shown in Table 2.

### Example 4: Granular agent having a coated core granular agent containing anhydrous glucose

In a tumbling granulator were charged 1,500 g of a core granular agent containing anhydrous glucose, and the temperature of the material was kept at 50°C. Coating of the core granular agent was performed using molten extremely hardened beef tallow and tabular crystals of secondary calcium phosphate dihydrate having a particle size of 10 µm in a proportion of 2 : 3 by weight in the same manner as in Example 1 to a coating ratio of 23 % by weight. Dissolution tests of the resulting granular agent were conducted in the same manner as in Example 1. Results obtained are shown in Table 2.

### Example 5: Granular agent having a coated core granular agent containing magnesium L-ascorbate phosphate

Coating of a core granular agent containing magnesium L-ascorbate phosphate was performed in the same manner as in Example 1 using extremely hardened beef tallow and tabular crystals of secondary calcium phosphate dihydrate having a particle size of 10 µm in a proportion of 2 : 3 by weight to a coating ratio of 23 % by weight. Dissolution tests of the resulting granular agent were conducted in the same manner as in Example 1. Results obtained are shown in Table 2.

### Example 6: Granular agent having a coated core granular agent containing choline chloride

Coating of a core granular agent containing choline chloride was performed in the same manner as in Example 1 using extremely hardened beef tallow and tabular crystals of secondary calcium phosphate dihydrate having a particle size of 10 µm in a proportion of 2 : 3 by weight to a coating ratio of 23 % by weight. Dissolution tests of the resulting granular agent were conducted in the same manner as in Example 1. Results obtained are shown in Table 2.

### Example 7: Granular agent having a coated core granular agent containing DL-methionine

Coating of a core granular agent containing DL-methionine was performed in the same manner as in Example 1 using extremely hardened beef tallow and tabular crystals of secondary calcium phosphate dihydrate having a particle size of 10 µm in a proportion of 2 : 3 by weight to a coating ratio of 20 % by weight. Dissolution tests of the resulting granular agent were conducted in the same manner as in Example 1. Results obtained are shown in Table 2.

### Example 8: Granular agent having a coated core granular agent containing L-tryptophan

Coating of a core granular agent containing L-tryptophan was performed in the same manner as in Example 1 using extremely hardened beef tallow and tabular crystals of secondary calcium phosphate dihydrate having a particle size of 10 µm in a proportion of 2:3 by weight to a coating ratio of 9 % by weight. Dissolution tests of the resulting granular agent were conducted in the same manner as in Example 1. Results obtained are shown in Table 2.

### Example 9: Granular agent having a coated core granular agent containing nicotinic acid

Coating of a core granular agent containing nicotinic acid was performed in the same manner as in Example 1 using extremely hardened beef tallow and tabular crystals of secondary calcium phosphate dihydrate having a particle size of 10 µm in a proportion of 2:3 by weight to a coating ratio of 26 % by weight. Dissolution tests of the resulting granular agent were conducted in the same manner as in Example 1. Results obtained are shown in Table 2.

### Example 10: Granular agent having a coated core granular agent containing betaine

Coating of a core granular agent containing betaine was performed in the same manner as in Example 1 using extremely hardened beef tallow and tabular crystals of secondary calcium phosphate dihydrate having a particle size of 10 µm in a proportion of 2:3 by weight to a coating ratio of 29 % by weight. Dissolution tests of the resulting granular agent were conducted in the same manner as in Example 1. Results obtained are shown in Table 2.

### Example 11: Granular agent having a coated core granular agent containing vitamin C

Coating of a core granular agent containing vitamin C was performed in the same manner as in Example 1 using extremely hardened beef tallow and tabular crystals of secondary calcium phosphate dihydrate having a particle size of 10 µm in a proportion of 2:3 by weight to a coating ratio of 26 % by weight. Dissolution tests of the resulting granular agent were conducted in the same manner as in Example 1. Results obtained are shown in Table 2.

### Example 12: Granular agent having a coated core granular agent containing glycine and alanine

Coating of a core granular agent containing glycine and DL-alanine was performed in the same manner as in Example 1 using extremely hardened beef tallow and tabular crystals of secondary calcium phosphate dihydrate having a particle size of 10 µm in a proportion of 3:4 by weight to a coating ratio of 22 % by weight. Dissolution tests of the resulting granular agent were conducted in the same manner as in Example 1. Results obtained are shown in Table 2.

**Table 2**

| Run | Physiologically Active Substance | Coating Material | Laminar Structure | Content (wt.%) | Dissolution Rate (%) | | |
|---|---|---|---|---|---|---|---|
| | | | | | Mimic-Rumen Solution | Sheep Rumen | Mimic-Abomasum Solution |
| Example 1 | LysHCl^{*1} | CaHPO₄·2H₂O | Yes | 49 | 17 | 20 | 42 |
| Example 2 | LysHCl^{*1} | L-Cystine | Yes | 49 | 24 | 25 | 43 |
| Example 3 | LysHCl^{*1} | D,L-Met^{*2} | Yes | 49 | 25 | 27 | 44 |
| Example 4 | Glucose | CaHPO₄·2H₂O | Yes | 57 | 17 | 11 | 55 |
| Example 5 | Magnesium L-ascorbate phosphate | CaHPO₄·2H₂O | Yes | 64 | 18 | 20 | 58 |
| Example 6 | Choline chloride | CaHPO₄·2H₂O | Yes | 45 | 17 | 20 | 49 |
| Example 7 | D,L-Met^{*2} | CaHPO₄·2H₂O | Yes | 65 | 12 | 18 | 47 |
| Example 8 | L-Tryptophan | CaHPO₄·2H₂O | Yes | 68 | 12 | 28 | 48 |
| Example 9 | Nicotinic acid | CaHPO₄·2H₂O | Yes | 51 | 10 | 16 | 50 |
| Example 10 | Betaine | CaHPO₄·2H₂O | Yes | 48 | 14 | 18 | 44 |
| Example 11 | Vitamin C | CaHPO₄·2H₂O | Yes | 58 | 19 | 25 | 51 |
| Example 12 | Glycine/DL-Alanine | CaHPO₄·2H₂O | Yes | 62 | 20 | 27 | 40 |
| Comparative Example 1 | LysHCl^{*1} | CaHPO₄·2H₂O (prismatic) | No | 49 | 94 | 98 | 86 |
| Comparative Example 2 | LysHCl^{*1} | Ca₃(PO₄)₂ (amorphous) | No | 49 | 71 | 75 | 80 |
| Comparative Example 3 | LysHCl^{*1} | CaCO₃ (amorphous) | No | 49 | 75 | 77 | 82 |
| Comparative Example 4 | LysHCl^{*1} | CaHPO₄·2H₂O (35 µm) | No | 49 | 35 | 41 | 40 |
| Comparative Example 5 | LysHCl^{*1} | CaHPO₄·2H₂O (2 µm) | Yes | 49 | 20 | 26 | 19 |

| | | | | | | | |
|---|---|---|---|---|---|---|---|
| Notes: *1: L-lysine hydrochloride; | | | | | | | |
| *2: DL-methionine; | | | | | | | |

### INDUSTRIAL APPLICABILITY

The present invention provides a feed or feed additive granular agent for ruminants having a strengthened coating layer and an excellent rumen bypass property, which agent comprises a core granular agent containing a physiologically active substance coated with a coating layer comprising (a) one or more substances (a first coating material) selected from the group consisting of a fatty acid having 12 to 22 carbon atoms or ester thereof, an animal or vegetable fat and fatty oil or a hardened animal or vegetable fat and fatty oil, having a melting point of 40°C or higher, and a wax having a melting point of 40°C or higher, and (b) tabular crystals of a substance (a second coating material) being sparingly water-soluble under a neutral condition but readily water-soluble under an acidic condition which have an average particle size of 5 to 30 µm, the first coating material and the second coating material being in a proportion of 3 : 2 to 1 : 3 by weight, and the coating layer having a laminar structure in which the tabular crystals are arranged in a laminated state, and a process for producing the same.

The granular agent for ruminants according to the present invention enables ruminants to directly absorb physiologically active substances useful to the ruminants and the use of the granular agent makes it possible to breed ruminants efficiently, which is very important industrially.

## Claims

1. A granular agent for ruminants, comprising a core granular agent containing a physiologically active substance coated with a coating layer comprising (a) a first coating material composed of one or more substances selected from the group consisting of a fatty acid having 12 to 22 carbon atoms or ester thereof, an animal or vegetable fat and fatty oil or a hardened animal or vegetable fat and fatty oil, having a melting point of 40°C or higher, and a wax having a melting point of 40°C or higher, and (b) a second coating material composed of tabular crystals of a substance being sparingly water-soluble under a neutral condition but readily water-soluble under an acidic condition which have an average particle size of 5 to 30 µm, said coating layer having a laminar structure in which said tabular crystals are arranged in a laminated state.

2. A granular agent for ruminants as claimed in Claim 1, wherein said first coating material and said second coating material are in a proportion of 3 : 2 to 1 : 3 by weight.

3. A granular agent for ruminants as claimed in Claim 1, wherein said coating layer is present in a coating ratio of 5 % by weight or more and 50 % by weight or less, based on the total weight of the granular agent.

4. A granular agent for ruminants as claimed in Claim 1, 2 or 3, wherein said physiologically active substance is at least one substance selected from the group consisting of amino acid, derivative of amino acid, vitamin, derivative of vitamin, enzyme, animal drug, hormone, carbohydrate, other nutrient source, microorganism and mineral.

5. A granular agent for ruminants as claimed in Claim 1, 2 or 3, wherein said second coating material is at least one substance selected from the group consisting of isoleucine, L-cystine, methionine, zinc phosphate octahydrate, and secondary calcium phosphate dihydrate.

6. A granular agent for ruminants as claimed in Claim 1, wherein said animal or vegetable fat and fatty oil is at least one substance selected from the group consisting of palm oil, beef tallow and porcine tallow.

7. A granular agent for ruminants as claimed in Claim 1, wherein said hardened animal or vegetable fat and fatty oil is at least one substance selected from the group consisting of hardened castor oil, hardened colza oil, hardened beef tallow, and extremely hardened beef tallow.

8. A granular agent for ruminants as claimed in Claim 1, wherein said wax having a melting point of 40°C or higher is at least one substance selected from the group consisting of bee wax, carnauba wax, and Japan wax.

9. A granular agent for ruminants as claimed in Claim 1, obtainable by a process which comprises providing a core granular agent containing said physiologically active substance, and coating said core granular agent with a coating layer comprising said first coating material and said second coating material in such a way as that said coating layer has laminar structure and that said tabular crystals are arranged in a laminated state in said coating layer.

10. A process for producing a granular agent for ruminants comprising a core granular agent containing a physiologically active substance and a coating layer, which comprises providing a core granular agent containing said physiologically active substance, and coating said core granular agent in a form in which said core granular agent is covered by a coating layer comprising
(a) a first coating material composed of one or more substances selected from the group consisting of a fatty acid having 12 to 22 carbon atoms or ester thereof, an animal or Vegetable fat and fatty oil or a hardened animal or vegetable fat and fatty oil, having a melting point of 40 °C or higher, and a wax having a melting point of 40 °C or higher, and
(b) a second coating material composed of tabular crystals of a substance being sparingly water-soluble under a neutral condition but readily water-soluble under an acidic condition which have an average particle size of 5 to 30 µm,
wherein said coating layer has a laminar structure in which said tabular crystals are arranged in a laminated state.

11. A process for producing a granular agent for ruminants as claimed in Claim 10,
wherein said first coating material and said second coating material are in a proportion of 3:2 to 1:3 by weight, and
wherein said coating layer is present in a coating ratio of 5 % by weight or more and 50 % by weight or less, based on the total weight of the granular agent.

12. A process for producing a granular agent for ruminants as claimed in Claim 10, wherein said physiologically active substance is at least one substance selected from the group consisting of amino acid, derivative of amino acid, vitamin, derivative of vitamin, enzyme, animal drug, hormone, carbohydrate, other nutrient source, microorganism and mineral.

13. A process for producing a granular agent for ruminants as claimed in Claim 10,
wherein said substance which is sparingly water-soluble under a neutral condition but readily water-soluble under an acidic condition is at least one substance selected from the group consisting of isoleucine, L-cystine, methionine, zinc phosphate octahydrate, and secondary calcium phosphate dihydrate.

14. A process for producing a granular agent for ruminants as claimed in Claim 10,
wherein said amimal or vegetable fat and fatty oil is at least one substance selected from the group consisting of palm oil, beef tallow and porcine tallow.

15. A process for producing a granular agent for ruminants as claimed in Claim 10,
wherein said hardened animal or vegetable fat and fatty oil is at least one substance selected from the group consisting of hardened castor oil, hardened colza oil, hardened beef tallow, and extremely hardened beef tallow.

16. A process for producing a granular agent for ruminants as claimed in Claim 10,
wherein said wax having a melting point of 40 °C or higher is at least one substance selected from the group consisting of bee wax, carnauba wax, and Japan wax.

17. A granular agent for ruminants comprising a core granular agent containing a physiologically active substance coated with a coating layer comprising
(a) a first coating material composed of one or more substances selected from the group consisting of a fatty acid having 12 to 22 carbon atoms or ester thereof, an animal or vegetable fat and fatty oil or a hardened animal or vegetable fat and fatty oil, having a melting point of 40 °C or higher, and a wax having a melting point of 40 °C or higher, and
(b) a second coating material composed of tabular crystals of a substance being sparingly water-soluble under a neutral condition but readily water-soluble under an acidic condition which have an average particle size of 5 to 30 µm,
wherein the dissolution rate of a physiologically active substance of said granular agent is not higher than 50 % when measured under the condition consisting of pH 7.1, 39 °C and 18 hours, and the dissolution rate of said substance of said granular agent is not lower than 20 % when measured under the condition consisting of pH 2, 39 °C and 18 hours.

18. A granular agent for ruminants as claimed in Claim. 17,
wherein said physiologically active substance is at least one substance selected from the group consisting of amino acid, derivative of amino acid, vitamin, derivative of vitamin, enzyme, animal drug, hormone, carbohydrate, other nutrient source, microorganism and mineral.

## Patentansprüche

1. Granuliertes Mittel für Wiederkäuer, umfassend einen eine physiologisch aktive Substanz enthaltenden Kern des granulierten Mittels, der mit einer Überzugsschicht beschichtet ist, die (a) ein erstes Überzugsmaterial, das aus einer oder mehr Substanzen zusammengesetzt ist, die aus der aus einer Fettsäure mit 12 bis 22 Kohlenstoffatomen oder deren Ester, einem tierischen oder pflanzlichen Fett und Fettöl oder gehärtetem tierischen oder pflanzlichen Fett und Fettöl mit einem Schmelzpunkt von 40°C oder darüber und einem Wachs mit einem Schmelzpunkt von 40°C oder darüber bestehenden Gruppe ausgewählt ist, und (b) ein zweites Überzugsmaterial, das aus Tafelkristallen einer unter neutralen Bedingungen wenig wasserlöslichen, aber unter sauren Bedingungen gut wasserlöslichen Substanz, die eine durchschnittliche Teilchengröße von 5 bis 30 µm aufweisen, zusammengesetzt ist, umfaßt, wobei die Überzugsschicht eine laminare Struktur aufweist, in der die Tafelkristalle schichtenförmig angeordnet sind.

2. Granuliertes Mittel für Wiederkäuer gemäß Anspruch 1, wobei das erste Überzugsmaterial und das zweite Überzugsmaterial in einem Gewichtsverhältnis von 3 : 2 bis 1 : 3 vorliegen.

3. Granuliertes Mittel für Wiederkäuer gemäß Anspruch 1, wobei die Überzugsschicht in einem Anteil von 5 Gew.-% oder mehr und 50 Gew.-% oder weniger, bezogen auf das Gesamtgewicht des granulierten Mittels, vorliegt.

4. Granuliertes Mittel für Wiederkäuer gemäß Anspruch 1, 2 oder 3, wobei die physiologisch aktive Substanz mindestens eine aus der aus Aminosäuren, Derivaten von Aminosäuren, Vitaminen, Derivaten von Vitaminen, Enzymen, Tierarzneimitteln, Hormonen, Kohlenhydraten, anderen Nährstoffquellen, Mikroorganismen und Mineralien bestehenden Gruppe ausgewählte Substanz ist.

5. Granuliertes Mittel für Wiederkäuer gemäß Anspruch 1, 2 oder 3, wobei das zweite Überzugsmaterial mindestens eine aus der aus Isoleucin, L-Cystin, Methionin, Zinkphosphat-octahydrat und sekundärem Calciumphosphat-dihydrat bestehenden Gruppe ausgewählte Substanz ist.

6. Granuliertes Mittel für Wiederkäuer gemäß Anspruch 1, wobei das tierische oder pflanzliche Fett und Fettöl mindestens eine aus der aus Palmöl, Rindertalg und Schweinetalg bestehenden Gruppe ausgewählte Substanz ist.

7. Granuliertes Mittel für Wiederkäuer gemäß Anspruch 1, wobei das gehärtete tierische oder pflanzliche Fett und Fettöl mindestens eine aus der aus gehärtetem Rizinusöl, gehärtetem Rapsöl, gehärtetem Rindertalg und extrem gehärtetem Rindertalg bestehenden Gruppe ausgewählte Substanz ist.

8. Granuliertes Mittel für Wiederkäuer gemäß Anspruch 1, wobei das Wachs mit einem Schmelzpunkt von 40°C oder darüber mindestens eine aus der aus Bienenwachs, Carnaubawachs und Japanwachs bestehenden Gruppe ausgewählte Substanz ist.

9. Granuliertes Mittel für Wiederkäuer gemäß Anspruch 1, das durch ein Verfahren erhältlich ist, das das Bereitstellen eines eine physiologisch aktive Substanz enthaltenden Kernes des granulierten Mittels und das Beschichten dieses Kerns des granulierten Mittels mit einer Überzugsschicht, die das erste Überzugsmaterial und das zweite Überzugsmaterial umfaßt, in einer Weise, daß die Überzugsschicht eine laminare Struktur aufweist und die Tafelkristalle in der Überzugsschicht schichtförmig angeordnet sind, umfaßt.

10. Verfahren zur Herstellung eines granulierten Mittels für Wiederkäuer, das einen Kern des granulierten Mittels umfaßt, der eine physiologisch aktive Substanz und eine Überzugsschicht umfaßt, wobei dieses Verfahren das Bereitstellen eines Kerns des granulierten Mittels, das die physiologisch aktive Substanz enthält, und das Beschichten des Kerns des granulierten Mittels in einer Weise umfaßt, daß der Kern des granulierten Mittels durch eine Überzugsschicht bedeckt ist, die
(a) ein erstes Überzugsmaterial, das aus einer oder mehr Substanzen zusammengesetzt ist, die aus der aus einer Fettsäure mit 12 bis 22 Kohlenstoffatomen oder deren Ester, einem tierischen oder pflanzlichen Fett und Fettöl oder einem gehärteten tierischen oder pflanzlichen Fett und Fettöl mit einem Schmelzpunkt von 40°C oder darüber und einem Wachs mit einem Schmelzpunkt von 40°C oder darüber bestehenden Gruppe ausgewählt sind, und
(b) ein zweites Überzugsmaterial, das aus Tafelkristallen einer unter neutralen Bedingungen wenig wasserlöslichen, aber unter sauren Bedingungen gut wasserlöslichen Substanz, die eine durchschnittliche Teilchengröße von 5 bis 30 µm aufweisen, zusammengesetzt ist, umfaßt,
wobei die Überzugsschicht eine laminare Struktur, in der die Tafelkristalle schichtenförmig angeordnet sind, aufweist.

11. Verfahren zur Herstellung eines granulierten Mittels für Wiederkäuer gemäß Anspruch 10, wobei das erste Überzugsmaterial und das zweite Überzugsmaterial in einem Gewichtsverhältnis von 3 : 2 bis 1 : 3 vorliegen und die Überzugsschicht in einem Anteil von 5 Gew.-% oder mehr und 50 Gew.-% oder weniger, bezogen auf das Gesamtgewicht des granulierten Mittels, vorliegt.

12. Verfahren zur Herstellung eines granulierten Mittels für Wiederkäuer gemäß Anspruch 10, wobei die physiologisch aktive Substanz mindestens eine aus der aus Aminosäuren, Derivaten von Aminosäuren, Vitaminen, Derivaten von Vitaminen, Enzymen, Tierarzneimitteln, Hormonen, Kohlenhydraten, anderen Nährstoffquellen, Mikroorganismen und Mineralien bestehenden Gruppe ausgewählte Substanz ist.

13. Verfahren zur Herstellung eines granulierten Mittels für Wiederkäuer gemäß Anspruch 10, wobei die Substanz, die unter neutralen Bedingungen wenig wasserlöslich, aber unter sauren Bedingungen leicht wasserlöslich ist, mindestens eine aus der aus Isoleucin, L-Cystin, Methionin, Zinkphosphat-octahydrat und sekundärem Calciumphosphat-dihydrat bestehenden Gruppe ausgewählte Substanz ist.

14. Verfahren zur Herstellung eines granulierten Mittels für Wiederkäuer gemäß Anspruch 10, wobei das tierische oder pflanzliche Fett und Fettöl mindestens eine aus der aus Palmöl, Rindertalg und Schweinetalg bestehenden Gruppe ausgewählte Substanz ist.

15. Verfahren zur Herstellung eines granulierten Mittels gemäß Anspruch 10, wobei das gehärtete tierische oder pflanzliche Fett und Fettöl mindestens eine aus der aus gehärtetem Rizinusöl, gehärtetem Rapsöl, gehärtetem Rindertalg und extrem gehärtetem Rindertalg bestehenden Gruppe ausgewählte Substanz ist.

16. Verfahren zur Herstellung eines granulierten Mittels für Wiederkäuer gemäß Anspruch 10, wobei das Wachs mit einem Schmelzpunkt von 40°C oder darüber mindestens eine aus der aus Bienenwachs, Carnaubawachs und Japanwachs bestehenden Gruppe ausgewählte Substanz ist.

17. Granuliertes Mittel für Wiederkäuer, welches einen Kern eines eine physiologisch aktive Substanz enthaltenden granulierten Mittels umfaßt, der mit einer Überzugsschicht beschichtet ist, die
(a) ein erstes Überzugsmaterial, das aus einer oder mehr Substanzen, die aus der aus einer Fettsäure mit 12 bis 22 Kohlenstoffatomen oder deren Ester, einem tierischen oder pflanzlichen Fett und Fettöl oder einem gehärteten tierischen oder pflanzlichen Fett und Fettöl mit einem Schmelzpunkt von 40°C oder darüber und einem Wachs mit einem Schmelzpunkt von 40°C oder darüber bestehenden Gruppe ausgewählt sind, und
(b) ein zweites Überzugsmaterial, das aus Tafelkristallen einer unter neutralen Bedingungen wenig wasserlöslichen, aber unter sauren Bedingungen gut wasserlöslichen Substanz, die einen durchschnittlichen Teilchendurchmesser von 5 bis 30 µm aufweisen, zusammengesetzt ist, umfaßt, wobei die Auflösungsrate der physiologisch aktiven Substanz des granulierten Mittels nicht höher als 50%, gemessen unter der Bedingung von pH 7,1, 39°C und 18 Stunden, und die Auflösungsrate der Substanz des granulierten Mittels nicht niedriger als 20%, gemessen unter der Bedingung von pH 2, 39°C und 18 Stunden, ist.

18. Granuliertes Mittel für Wiederkäuer gemäß Anspruch 17, wobei die physiologisch aktive Substanz mindestens eine aus der aus Aminosäuren, Derivaten von Aminosäuren, Vitaminen, Derivaten von Vitaminen, Enzymen, Tierarzneimitteln, Hormonen, Kohlenhydraten, anderen Nährstoffquellen, Mikroorganismen und Mineralien bestehenden Gruppe ausgewählte Substanz ist.

## Revendications

1. Agent granulaire pour ruminants, comprenant un agent granulaire formant noyau contenant une substance physiologiquement active, enrobé d'une couche d'enrobage comprenant (a) une première matière d'enrobage composée d'une ou plusieurs substances sélectionnées dans le groupe constitué par un acide gras ayant de 12 à 22 atomes de carbone ou un ester de ce dernier, une graisse et huile grasse animale ou végétale ou une graisse et huile grasse animale ou végétale durcie, ayant un point de fusion supérieur ou égal à 40°C et une cire ayant un point de fusion supérieur ou égal à 40°C et (b) une deuxième matière d'enrobage composée de cristaux tabulaires d'une substance faiblement soluble dans l'eau dans un environnement neutre, mais facilement soluble dans l'eau dans un environnement acide, qui ont une dimension moyenne de particule de 5 à 30 µm, ladite couche d'enrobage ayant une structure laminaire dans laquelle lesdits cristaux tabulaires sont disposés à un état laminé.

2. Agent granulaire pour ruminants selon la revendication 1, où ladite première matière d'enrobage et ladite deuxième matière d'enrobage sont dans une proportion de 3:2 à 1:3 en poids.

3. Agent granulaire pour ruminants selon la revendication 1, où ladite couche d'enrobage est présente pour un rapport d'enrobage de 5 % en poids ou plus et de 50 % en poids ou moins, par rapport au poids total de l'agent granulaire.

4. Agent granulaire pour ruminants selon la revendication 1, 2 ou 3, où ladite substance physiologiquement active est au moins une substance sélectionnée dans le groupe constitué par un acide aminé, un dérivé d'acide aminé, une vitamine, un dérivé de vitamine, une enzyme, un médicament pour animaux, une hormone, un glucide, une autre source nutritive, un micro-organisme et un minéral.

5. Agent granulaire pour ruminants selon la revendication 1, 2 ou 3, où ladite deuxième matière d'enrobage est au moins une substance sélectionnée dans le groupe constitué par l'isoleucine, la L-cystéine, la méthionine, le phosphate de zinc octahydraté et le phosphate de calcium secondaire dihydraté.

6. Agent granulaire pour ruminants selon la revendication 1, où ladite graisse et huile grasse animale ou végétale est au moins une substance sélectionnée dans le groupe constitué par l'huile de palme, le suif de boeuf et le saindoux de porc.

7. Agent granulaire pour ruminants selon la revendication 1, où ladite graisse et huile grasse animale ou végétale durcie est au moins une substance sélectionnée dans le groupe constitué par l'huile de ricin durcie, l'huile de colza durcie, le suif de boeuf durci et le suif de boeuf extrêmement durci.

8. Agent granulaire pour ruminants selon la revendication 1, où ladite cire ayant un point de fusion supérieur ou égal à 40°C est au moins une substance sélectionnée dans le groupe constitué par la cire d'abeilles, la cire de carnauba et la cire du Japon.

9. Agent granulaire pour ruminants selon la revendication 1, susceptible d'être obtenu par un procédé qui comprend la mise à disposition d'un agent granulaire formant noyau contenant ladite substance physiologiquement active et l'enrobage dudit agent granulaire formant noyau par une couche d'enrobage comprenant ladite première matière d'enrobage et ladite deuxième matière d'enrobage de façon telle que ladite couche d'enrobage a une structure laminaire et que lesdits cristaux tabulaires sont disposés à un état laminé dans ladite couche d'enrobage.

10. Procédé de production d'un agent granulaire pour ruminants comprenant un agent granulaire formant noyau contenant une substance physiologiquement active et une couche d'enrobage, lequel comprend la mise à disposition d'un agent granulaire formant noyau contenant ladite substance physiologiquement active et l'enrobage dudit agent granulaire formant noyau sous une forme où ledit agent granulaire formant noyau est couvert par une couche d'enrobage comprenant
(a) une première matière d'enrobage composée d'une ou plusieurs substances sélectionnées dans le groupe constitué par un acide gras ayant de 12 à 22 atomes de carbone ou un ester de ce dernier, une graisse et huile grasse animale ou végétale ou une graisse et huile grasse animale ou végétale durcie ayant un point de fusion supérieur ou égal à 40°C, et une cire ayant un point de fusion supérieur ou égal à 40°C et
(b) une deuxième matière d'enrobage composée de cristaux tabulaires d'une substance faiblement soluble dans l'eau dans un environnement neutre, mais facilement soluble dans l'eau dans un environnement acide, et qui ont une dimension moyenne de particule de 5 à 30 µm,
où ladite couche d'enrobage a une structure laminaire dans laquelle lesdits cristaux tabulaires sont disposés à un état laminé.

11. Procédé de production d'un agent granulaire pour ruminants selon la revendication 10, où ladite première matière d'enrobage et ladite deuxième matière d'enrobage sont dans une proportion de 3:2 à 1:3 en poids et où ladite couche d'enrobage est présente pour un rapport d'enrobage de 5 % en poids ou plus et de 50 % en poids ou moins, par rapport au poids total de l'agent granulaire.

12. Procédé de production d'un agent granulaire pour ruminants selon la revendication 10, où ladite substance physiologiquement active est au moins une substance sélectionnée dans le groupe constitué par un acide aminé, un dérivé d'acide aminé, une vitamine, un dérivé de vitamine, une enzyme, un médicament pour animaux, une hormone, un glucide, une autre source nutritive, un micro-organisme et un minéral.

13. Procédé de production d'un agent granulaire pour ruminants selon la revendication 10, où ladite substance faiblement soluble dans l'eau dans un environnement neutre mais facilement soluble dans l'eau dans un environnement acide est au moins une substance sélectionnée dans le groupe constitué par l'isoleucine, la L-cystéine, la méthionine, le phosphate de zinc octahydraté et le phosphate de calcium secondaire dihydraté.

14. Procédé de production d'un agent granulaire pour ruminants selon la revendication 10, où ladite graisse et huile grasse animale ou végétale est au moins une substance sélectionnée dans le groupe constitué par l'huile de palme, le suif de boeuf et le saindoux de porc.

15. Procédé de production d'un agent granulaire pour ruminants selon la revendication 10, où ladite graisse et huile grasse animale ou végétale durcie est au moins une substance sélectionnée dans le groupe constitué par l'huile de ricin durcie, l'huile de colza durcie, le suif de boeuf durci et le suif de boeuf extrêmement durci.

16. Procédé de production d'un agent granulaire pour ruminants selon la revendication 10, où ladite cire ayant un point de fusion supérieur ou égal à 40°C est au moins une substance sélectionnée dans le groupe constitué par la cire d'abeilles, la cire de carnauba et la cire du Japon.

17. Agent granulaire pour ruminants comprenant un agent granulaire formant noyau contenant une substance physiologiquement active, enrobé d'une couche d'enrobage comprenant
(a) une première matière d'enrobage composée d'une ou plusieurs substances sélectionnées dans le groupe constitué par un acide gras ayant de 12 à 22 atomes de carbone ou un ester de ce dernier, une graisse et huile grasse animale ou végétale ou une graisse et huile grasse animale ou végétale durcie ayant un point de fusion supérieur ou égal à 40°C, et une cire ayant un point de fusion supérieur ou égal à 40°C et
(b) une deuxième matière d'enrobage composée de cristaux tabulaires d'une substance faiblement soluble dans l'eau dans un environnement neutre, mais facilement soluble dans l'eau dans un environnement acide, qui ont une dimension moyenne de particule de 5 à 30 µm,
où le taux de dissolution d'une substance physiologiquement active dudit agent granulaire est inférieur ou égal à 50 % lorsqu'il est mesuré dans des conditions réunissant pH de 7,1, 39°C et 18 h, et le faux de dissolution de ladite substance dudit agent granulaire est supérieur ou égal à 20 % lorsqu'il est mesuré dans des conditions réunissant pH de 2, 39°C et 18 h.

18. Agent granulaire pour ruminants selon la revendication 17, où ladite substance physiologiquement active est au moins une substance sélectionnée dans le groupe constitué par un acide aminé, un dérivé d'acide aminé, une vitamine, un dérivé de vitamine, une enzyme, un médicament pour animaux, une homone, un glucide, une autre source nutritive, a micro-organisme et un minéral.
